# EUROPEAN PATENT SPECIFICATION

(11) **EP 1 750 582 B1**
(45) Date of publication and mention of the grant of the patent: **18.03.2009**
(21) Application number: 05754689.7
(22) Date of filing: 03.06.2005
(51) Int. Cl.: A61B 5/22, A61B 5/0488, A61B 5/11

(54) **MEDICAL DEVICE ADAPTED TO THE MONITORING OF LIMB MUSCLE BEHAVIOUR IN PATIENTS**
MEDIZINPRODUKT ZUR ÜBERWACHUNG DES GLIEDMASSEN-MUSKELVERHALTENS VON PATIENTEN
APPAREIL MEDICAL POUR LA SURVEILLANCE DU COMPORTEMENT DES MUSCLES DES EXTREMITES

(30) Priority: 04.06.2004 EP 04447136
(43) Date of publication of application: 14.02.2007
(73) Proprietor: UNIVERSITE LIBRE DE BRUXELLES, 1050 Bruxelles (BE); FCS Control Systems, 1117 ZJ Schiphol (NL)
(72) Inventor: MANTO, Mario, B-1440 Braine-le-Château (BE); VAN DER LINDE, Richard, NL-2662 AN BERGSCHENHOEK (NL); LAMMERTSE, Piet, NL-1181 SW AMSTELVEEN (NL)
(74) Representative: Van Malderen, Joëlle
(86) International application number: PCT/BE2005/000090
(87) International publication number: WO 2005/117705

(56) References cited:
- DE-A- 4 425 256
- US-A- 5 012 820
- US-A- 5 513 651
- US-A- 6 155 993
- US-A1- 2002 183 655

## Description

### Field of the invention

The present invention is related to a medical device which is preferably portable and a method for the analysis and/or the monitoring of the behaviour of limb muscles, including muscle fibres and motor units, in a patient.

In particular, the present invention is related to a medical device and method for dynamic analysis and/or monitoring of voluntary and involuntary movements in upper or lower limbs of a patient.

### State of the art

Despite recent advances in the management of patients presenting a central and/or peripheral nervous system lesion, neurologists and therapists still evaluate voluntary and involuntary movements of upper and lower limbs, and namely movements of the wrist joint, clinically in a subjective manner depending on the examiner. As a consequence, such clinical evaluation lacks reliability.

The interest of a medical device or instrument which could help the examiner in his diagnosis is therefore obvious.
Prior art document US 2002/183655 discloses a portable intelligent stretching device for use by patients suffering from spastic and contractured joints and limbs. The intelligent stretching device has a motor and a motor shaft for rotating the joint or limb. The variable velocity and stretch distance of the device is determined by a torque sensor on the joint or limb that communicates information to a controller which subsequently instructs the motor as to the variable velocity and stretch distance. During movement of the limb and joint, the joint angle, resistance torque and Electromyogram (EMG) signals from the soleus, gastronemius and tibialis anterior muscles are recorded. The EMG signals are recorded via electrodes attached to these muscles and subsequently connected to the computer for recordation and further analysis. The electrodes emit electronic signals to the computer corresponding to those emitted by the muscles. The computer can then communicate with the controller to increase or decrease the limits of the range of motion or the variable velocity based upon the information provided by the electrodes to better tailor the device to a specific patient.

### Aims of the invention.

The present invention aims to provide a medical device and method for a systematical analysis and/or monitoring limb movements in a patient.

The present invention aims to provide a medical device and method giving reliable data.

The present invention aims to provide a medical device and method easy to use or to implement.

The present invention aims to provide a medical device and method which provide rapidly interpretable data by a trained person.

### Summary of the invention

The present invention is related to a medical device, which is preferably portable, for the analysis and/or the monitoring of limb muscle behaviour in a patient, preferably a simultaneous analysis of all the tested limb muscles during voluntary and/or involuntary mechanical movements of the patient.
According to the invention, the limb corresponds to a leg, an arm or the neck of a patient and the medical device comprises :
- a moving unit adapted to receive a first part of said leg, arm or neck, corresponding to the foot, to the foreleg, to the forearm, to the hand and/or to the head, said moving unit presenting at least one degree of freedom according to which the moving unit may move;
- a haptic device adapted to receive a second part of said leg, said arm or said neck, corresponding to the foreleg, to the upperleg, to the upperarm, to the forearm and/or to the neck, respectively, and to measure in the form of an analogical signal the mechanical response and/or dynamic response of said second part to a movement of the moving unit;
- a set of electromyographic electrodes able to measure in the form of an analogical signal the electrical activities in the muscle(s) of said leg, arm or neck, respectively;
- an analogical signal acquisition unit able to acquire the analogical signals from the electromyographic electrodes and the haptic device and converting said analogical signals into digital signals;
- a controller able to process said digital signals and to control the moving unit.

Similarly to the moving unit, the haptic device may also comprise at least one degree of freedom.

In the present description, it is meant by "muscle" the muscle as whole and/or one or more muscle fibres and/or one or more motor units of said muscle.

Preferably, the moving device in operating conditions is driven by a motor under the control of the controller.

Preferably, the electromyographic electrodes comprise needle electrodes and/or surface electrodes and/or microelectrodes. Preferably, the electromyographic electrodes are needle electrodes and/or surface electrodes and/or microelectrodes.

Preferably, the medical device according to invention further comprises an amplification system for amplifying the analogical signals measured by the electrodes.

Preferably, the controller is configured so as to control or maintain in operating conditions in direction and in intensity the driving force of the motor and thereby to control or maintain in direction and in intensity the motion of the moving unit.

The present medical device is also adapted for passive monitoring of spontaneous patient motions.

Advantageously, the moving unit of the present medical device presents one rotation axis around which the moving unit is able to rotate relatively to the haptic device, preferably with a rotation angle comprised between at least about + 45° and at least about -45° and a defined maximal rotation velocity.

Preferably, said maximum rotation velocity is about 300°/sec.

Advantageously also, the moving unit has a position accuracy of about 0.1 mm. According to a preferred embodiment, the present device further comprises a drug(s) delivery device to the patient.

A feedback, which can be passive, static or dynamic, of patient force can be used for the motion of the device.

Furthermore, on some embodiments, the device can be used to dynamically react to patient forces.

The present invention also concerns a method for the analysis and/or the monitoring of the behaviour of a limb muscle using said portable medical device, said limb being either the leg, the arm or the neck of a patient. Said method comprises the following steps of :
- placing the first part of the limb (the foot, the hand, or the neck respectively) of the patient on the moving unit;
- placing the second part of the limb (the foot, the foreleg, the forearm, the hand or the head respectively) on the haptic device in such a way that the main axis of the ankle, the wrist, the knee or the neck, respectively coincides with the main axis of the motor;
- placing the electromyographic electrodes at different positions in and/or on the muscle of the first part of the limb (the foot, the foreleg, the forearm, the hand or the head respectively);
- applying under the control of the controller a force of precise direction and intensity to the motor so as to move with a force of precise direction and intensity the moving unit and first part of the limb (the foot, the hand or the neck respectively) placed thereon;
- measuring the mechanical response (direction and intensity of the movements) of second part of the limb (the foreleg, the forearm, the hand or the head, respectively) by means of the haptic device;
- measuring the electrical response of the first part of the limb (the foot, the foreleg, the forearm, the hand or the neck) by means of the electromyographic electrodes at different locations along said part of the limb (foot, foreleg, forearm hand or neck, respectively) ;
- acquiring said mechanical and electrical responses in the form of analogical signals by means of .the acquiring unit;
- converting said analogical signals into digital signals;
- processing by means of the controller said digital signals.

The method may also comprise the step of applying under the control of the controller a displacement of precise direction and amplitude to the moving unit and the first part of the limb (the foot, the hand or the neck), respectively placed thereon, and measuring the resulting force at the second part of the limb (foreleg, forearm, hand or head) by means of the haptic device.

As already specified hereabove, it is meant by "muscle" the muscle as a whole and/or the muscle fibres and/or the motor units of said muscle.

Preferably, said analogical signals are acquired with respect to time and processed so as to have a monitoring of limb movements along time.

The present invention is also related to the use of the present medical device and/or the present method as a tool for monitoring voluntary and involuntary limb movements in a patient.

The present invention also concerns the use of the present medical device and/or the present method as an indirect tool providing physical data for evaluating the stage of recovery of a patient.

Another object of the present invention is the use of the present medical device and/or the present method for investigating the effects of drugs in a patient.

It has to be noted that the medical device and method according to the present invention provide data corresponding to intermediate results about the limb muscle behaviour which do not on their own enable a decision to be made by the doctor or surgeon on the diagnosis and the treatment necessary.

### Short description of the drawings

Fig. 1 represents an embodiment of a medical device as used for monitoring the behaviour of arm muscles and motor units according to the present invention.

Fig. 2a and Fig. 2b present respectively a side view and a top view of a medical device (moving unit + haptic device) as used in a preferred embodiment of the present medical device.

### Detailed description of the invention

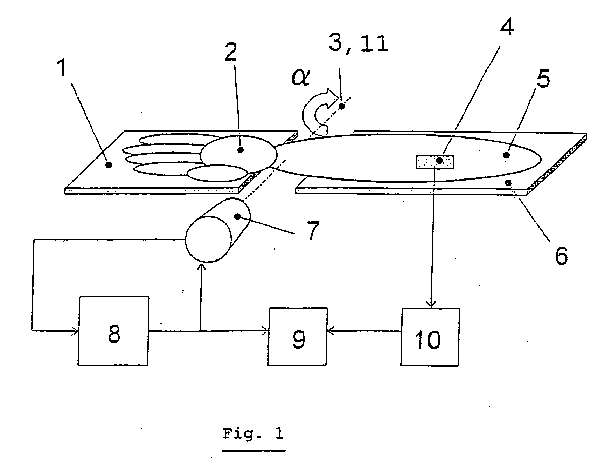

As illustrated in Fig. 1, the medical device according to the invention is a portable medical device comprising:
- moving unit 1 able to move according to at least one degree of freedom;
- a motor 7 for driving the moving unit 1, said motor being provided with a main rotation axis 3;
- a haptic device 6 having at least one degree of freedom and being able to measure by tactile contact of an object using haptic technology the mechanical motions, in amplitude and orientation, of said object;
- a set of electromyographic electrodes 4;
- a signal amplification unit 10;
- a signal acquisition unit 9;
- and a controller 8 for driving the motor 7 with calibrated motions and having signal processing capacities.

In the present embodiment, the moving unit presents a rotation axis 11 which coincides with the rotation axis 3 of the motor 7. The moving unit is thus able to rotate around its axis 11 relatively to the haptic device 6, with a precise rotation angle α and at a precise rotation speed or velocity (under the control of the controller 8).

The moving unit 1 could also be able to be translated. Possibly also, the haptic device 6 could be able to be rotated and/or translated.

The haptic device 6 of the present medical device is able to measure forces using the haptic technology.

The principle of said medical device is to induce a mechanical perturbation, preferably via the moving unit 1, in an upper limb or lower limb of a patient and to measure the mechanical and electrical response of the muscles of said upper or lower limb to said perturbations.

The medical device is adapted to the study of a limb muscle as a whole and/of of its muscle fibres and/or of its motor units.

In operating conditions, the method for monitoring the movements of a limb, such as an arm as illustrated in Fig. 1, using the present medical device, comprises the following steps:
- the arm is placed relatively to the medical device such that the hand 2 lies on the moving unit 1 and the forearm 5 lies on the haptic device 6, with the main axis of the wrist coinciding with the main axis 3 of the motor 7;
- once the arm is correctly placed, arranging the electromyographic electrodes 4 at different locations along the arm, and preferably along the forearm 5, in the muscles or on the skin of the patient;
- ordering to the motor 7, via the controller 8, to drive the moving unit 1, and thus the hand 2, following a movement (or a pattern of (different) movements) with precise direction and amplitude;
- measuring by means of the haptic device 6 the mechanical response in amplitude, orientation, and force of the forearm 5 of the patient;
- simultaneously measuring by means of the electrodes 4 the electrical response (electrical activities) at different locations along the arm, and preferably along the forearm 5;
- acquiring with the acquisition the electrical and mechanical responses in the form of analogical signals;
- converting said analogical signals into digital signals;
- processing by means of the controller 8 said digital signals so as to provide data about the movements of the arm of the patient which could be then interpreted by a trained person.

It should be noted that the medical device may comprise 4 different electromyographic electrodes able to be inserted in four forearm muscles, flexor carpi radialis, extensor carpi radialis, brachio-radialis, and flexor carpi ulnaris. (Active) surface electrodes could be affixed at the skin at the level of the flexor carpi radialis and extensor carpi radialis muscles.

Possibly, the axis of the motor can be moved easily and quickly in more than one plane.

One advantage of the present medical device is that it is so configured and dimensioned that it is portable and can be used on the bedside of a patient, with a patient in different possible positions: horizontal, vertical, or seated position. The medical device is thus very comfortable for the patient and could even be used in extreme conditions (emergency situations for example).

The medical device and method according to the invention offer a wide range of applications.

It is possible to identify and analyse the discharges of individual muscle fibres or single motor units in different conditions of impedance of the foreleg, forearm or neck joint.

The present medical device can be combined with a source of external stimuli (preferably electrical or magnetic stimuli), for example over a region of the central nervous system (preferably the motor cortex or the spinal cord) or the peripheral nervous system (preferentially a plexus), in order to analyse the control mechanism by the nervous system on the behaviour of motor units and muscle fibres during mechanical stretches of a muscle.

As already disclosed above, the present medical device and method could be used as a tool to investigate the mechanism of neurological control of wrist movements in patients presenting a central and/or peripheral nervous system lesion.

For example also, the present medical device and method could be used to analyse a pathological hand tremor through the characterisation of its frequency band and its muscles coherence in different impedance conditions an in different conditions of inertia. This analysis could provide indirect information useful in the establishment of a neurological differential diagnosis.

The present medical device can be used as a tool for the clinician which may help him to establish a differential diagnosis of psychogenic tremor.

The present medical device and method could also be used to test the effect of candidate drugs on limb tremor, in particular hand tremor, and for example in essential hand tremor or hand tremor related to Parkinsons disease.

Another possible application of the present medical device and method is their use as a tool to precisely evaluate the mechanism of recovery of cerebellar dysmetria in human and then to determine the stage of recovery reached by a patient following a cerebellar stroke. In that sense, the present medical device and method could indirectly help doctors to improve neurological rehabilitation of patients.

The present medical device and patient could also be used for objectively evaluating the tone of wrist joint during neurosurgery procedures in patients suffering from Parkinson's disease. From that point of view, the present medical device and method could indirectly help doctors to perform a more efficient deep brain stimulation procedure (for example neurostimulation of subthalamic nuclei).

The present medical device can also be used as a tool to analyse hand control in patients after grafting of a hand.

The present medical device could also be used to test prosthesis or orthesis, such as thermoformable prosthesis and/or orthesis.

Rehabilitation of the limb of a patient after an accident is also a possible application of the present medical device.

As illustrated above, one of the main advantage of the present medical device comparatively to the prior art is to provide objective data.

However, it should be understood that the medical device and method according to the present invention only provide data corresponding to intermediate results about the limb muscle behaviour which do not on their own enable a decision to be made by the doctor or surgeon on the treatment necessary.

However, the device according to the invention, contrary to the known devices of the state of the art, can combine the analysis of surface EMG activity and intramuscular activity, coupling with the analysis of motor unit of muscles and/or muscle fibres without any induced mechanical movements upon the limbs of the patient.

In addition, said.device.could be used for the analysis and/or the monitoring of various oscillatory, voluntary or involuntary movements of a patient.

Furthermore, the device according to the invention could be used for the analysis and/or the monitoring of the effects of high-frequency vibrations upon limb muscles behaviour, especially upon the motor units of these muscles or muscle fibres from a different muscle, especially contiguous muscular fibres.

Advantageously, the device could be used for maintaining a constant strength at a pre-determinated position of the arm, leg or neck of the patient and therefore for recording their intramuscular EMG associated activities. This analysis is particularly useful for recording movements induced by a motor unit of a muscle in different positions following automatic movements from one position to another.

The medical device according to the invention is particularly ergonomically adapted to the patient limbs, such as his neck, hand, arm, foot or leg.

Finally, a retro-controlled loop could be introduced in the control of the various elements of the device according to the analysis and/or monitoring of intramuscular activities in real time.

The device according to the invention allows also an instant adaptation of oscillation frequency, according to the limb muscle behaviour.

Another possible application of the present medical device is tetanic stimulation. More precisely, the present medical device may be used to analyze the frequencies of muscular contractions (detected by means of either active electrodes placed on the skin, or intra-muscular electrodes, or both of them). With these frequencies, one can study the behaviour of motor units known as slow motor units can be studied, and this behaviour can be compared to the behaviour of motor units known as rapid motor units.

Furthermore, the present medical device allows instantaneous calculation of a rigidity index from the torque/position curves by linear regression measurements. This technical characteristic correspond to a substantial advantage in the perspective of a pharmacological evaluation of new drugs interacting with the central nervous system and of the search for a possible change of articulation impedance induced by a drug. This rigidity is very rapidly calculated.

Finally, the device according to the invention is adapted to identify the frequencies of the motor units of muscles and identify which dynamic perturbation could induce various force according to the position of the arm, leg or neck.

The device applies also for detecting simultaneously voluntary or involuntary movements and articular rigidity in patients suffering from dystonic movements with extra-pyramidal rigidity.

According to a last embodiment of the present invention, the device according to the invention could be programmed in order to induce various successive mobilisation at various oscillation frequencies, according to a sequential rehabilitation programme in the absence of any external influence.

### Example of a particular embodiment:

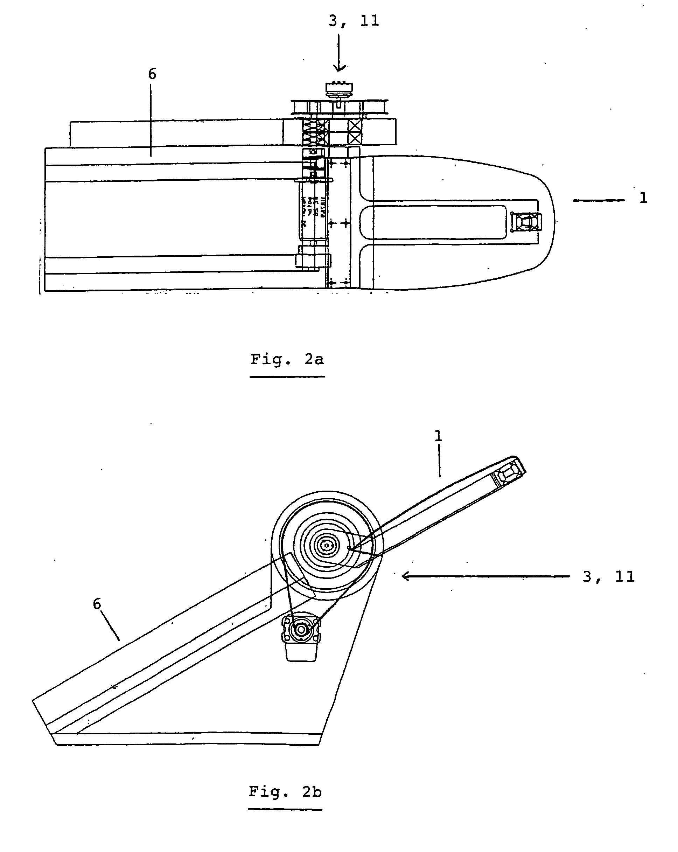

According to a preferred embodiment of the invention, the medical device presents the following technical characteristics:
- 4 differential amplifiers with a gain of 1000 (as for example the ones produced by the Xltek company, Canada);
- 4 sterile concentric needle electrodes 30G (for example as the ones sold by the Medtronic company, (Skovlunde, Denmark));
- two surface active electrodes with preamplification and filters incorporated therein (gain of 1000; filter 20-450 Hz, for example as the ones produced by Delsys DE-2.3) ;
- 1 analog acquisition unit;
- 1 personal computer; and
- a moving unit coupled to a haptic device as illustrated in Fig. 2a and 2b, with one degree of freedom with effort return or force feedback (rotation axis with numerical reference 11) to generate an arbitrary dynamic behaviour.

The characteristics of said moving unit were the following:
- position accuracy: 0.1 mm at the level of fingers extremities on the haptic device;
- nominal torque: 4 Nm;
- peak torque: 20 Nm;
- maximal rotation velocity: 300 deg/sec
- range of motion: from +45 to -45 degrees.

## Claims

1. A medical device for monitoring limb muscle behaviour in a patient, said limb corresponding to a leg, an arm or the neck, said medical device comprising:
- a moving unit (1) adapted to receive a first part of a limb corresponding to a second part of a limb, said moving unit (1) presenting at least one degree of freedom according to which the moving unit (1) may move;
- a haptic device (6) adapted to receive a second part of the limb corresponding to the second part of the limb respectively, and to measure in the form of an analogical signal the mechanical response of said second part to a movement of the moving unit (1);
- a set of electromyographic electrodes (4) able to measure in the form of an analogical signal the electrical activities in the muscle(s) of the limb (the leg, the arm, or the neck, respectively);
- an analogical signal acquisition unit (9) able to acquire the analogical signals from the electromyographic electrodes and the haptic device (6) and converting said analogical signals into digital signals;
- a controller (8) able to process said digital signal and to control the moving unit (1).

2. The medical device according to claim 1, **characterized in that** the moving device is driven by a motor (7) under the control of the controller (8).

3. The medical device according to .claim 1 or 2, **characterized in that** the electromyographic electrodes are needle electrodes and/or surface electrodes.

4. The medical device according to any one of the preceding claims, **characterized in that** it comprises four electromyographic needle electrode and two electromyographic surface active electrodes.

5. The medical device according to claim 1 or 2, **characterized in that** the electromyographic electrodes are microelectrodes.

6. The medical device according to any one of the preceding claims, **characterized in that** the moving unit (1) presents at least one rotation axis (11) around which the moving unit (1) is able to rotate relatively to the haptic device (6) with a rotation angle (α) comprised between about + 45 ° and about - 45 ° and a defined maximal rotation velocity.

7. The medical device according to claim 6, **characterized in that** the maximum rotation velocity of the moving unit (1) is about 300°/sec.

8. The medical device according to claim 6 or 7, **characterized in that** the moving unit (1) has a position accuracy of about 0.1 mm.

9. The medical device according to any one of the preceding claims, **characterized in that** it further comprises an amplification system for amplifying the analogical signals measured by the electrodes (4).

10. The medical device according to claim 1 or 2, **characterized in that** the controller maintains in direction and in intensity the driving force of the motor (7) and thereby in direction and in intensity the motion of the moving unit (1).

## Patentansprüche

1. Medizinische Vorrichtung zum Überwachen des Verhaltens der Gliedmaßenmuskeln bei einem Patienten, wobei das Glied einem Bein, einem Arm oder dem Hals entspricht, wobei die medizinische Vorrichtung folgendes umfasst:
- eine bewegliche Einheit (1), die dazu geeignet ist, einen ersten Teil eines Glieds aufzunehmen, der einem zweiten Teil eines Glieds entspricht, wobei die bewegliche Einheit (1) mindestens einen Freiheitsgrad aufweist, demgemäß sich die bewegliche Einheit (1) bewegen kann;
- eine haptische Vorrichtung (6), die dazu geeignet ist einen zweiten Teil des Glieds aufzunehmen, das jeweils dem zweiten Teil des Glieds entspricht, und in Form eines analogen Signals die mechanische Reaktion des zweiten Teils auf eine Bewegung der beweglichen Einheit (1) zu messen;
- einen Satz elektromyographischer Elektroden (4), die in der Lage sind, in Form eines analogen Signals die elektrischen Aktivitäten des Muskels bzw. der Muskeln des Glieds (jeweils des Beins, Arms oder Halses) zu messen;
- eine Einheit (9) zum Erfassen analoger Signale, die in der Lage ist, die analogen Signale von den elektromyographischen Elektroden und der haptischen Vorrichtung (6) zu erfassen und die analogen Signale in digitale Signale umzuwandeln;
- ein Steuergerät (8), das in der Lage ist, die digitalen Signale zu verarbeiten und die bewegliche Einheit (1) zu steuern.

2. Medizinische Vorrichtung nach Anspruch 1, **dadurch gekennzeichnet, dass** die bewegliche Vorrichtung von einem Motor (7) unter der Kontrolle des Steuergeräts (8) angetrieben wird.

3. Medizinische Vorrichtung nach Anspruch 1 oder 2, **dadurch gekennzeichnet, dass** die elektromyographischen Elektroden Nadelelektroden und/oder Oberflächenelektroden sind.

4. Medizinische Vorrichtung nach einem der vorhergehenden Ansprüche, **dadurch gekennzeichnet, dass** sie vier elektromyographische Nadelelektroden und zwei elektromyographische oberflächenaktive Elektroden umfasst.

5. Medizinische Vorrichtung nach Anspruch 1 oder 2, **dadurch gekennzeichnet, dass** die elektromyographischen Elektroden Mirkoelektroden sind.

6. Medizinische Vorrichtung nach einem der vorhergehenden Ansprüche, **dadurch gekennzeichnet, dass** die bewegliche Einheit (1) mindestens eine Drehachse (11) aufweist, um welche sich die bewegliche Einheit (1) im Verhältnis zu der haptischen Vorrichtung (6) in einem Drehwinkel (α), der zwischen ungefähr +45° und ungefähr -45° liegt, und mit einer bestimmten maximalen Drehgeschwindigkeit drehen kann.

7. Medizinische Vorrichtung nach Anspruch 6, **dadurch gekennzeichnet, dass** die maximale Drehgeschwindigkeit der beweglichen Einheit (1) ungefähr 300°/s beträgt.

8. Medizinische Vorrichtung nach Anspruch 6 oder 7, **dadurch gekennzeichnet, dass** die bewegliche Einheit (1) eine Lagegenauigkeit von ungefähr 0,1 mm aufweist.

9. Medizinische Vorrichtung nach einem der vorhergehenden Anspruch, **dadurch gekennzeichnet, dass** sie ferner ein verstärkungssystem umfasst zum Verstärken der analogen Signale, die von den Elektroden (4) gemessen werden.

10. Medizinische Vorrichtung nach Anspruch 1 oder 2, **dadurch gekennzeichnet, dass** das Steuergerät in Richtung und Stärke die Antriebskraft des Motors (7) und somit in Richtung und Stärke die Bewegung der beweglichen Einheit (1) aufrechterhält.

## Revendications

1. Dispositif médical pour surveiller le comportement des muscles d'un membre chez un patient, ledit membre correspondant à une jambe, à un bras ou au cou, ledit dispositif médical comprenant :
- une unité mobile (1) adaptée pour recevoir une première partie d'un membre correspondant à une deuxième partie d'un membre, ladite unité mobile (1) présentant au moins un degré de liberté selon lequel l'unité mobile (1) peut se déplacer ;
- un dispositif haptique (6) adapté pour recevoir une deuxième partie du membre correspondant à la deuxième partie du membre, respectivement, et pour mesurer sous la forme d'un signal analogique la réponse mécanique de ladite deuxième partie à un mouvement de l'unité mobile (1) ;
- un ensemble d'électrodes électromyographiques (4) capables de mesurer sous la forme d'un signal analogique les activités électriques dans le(s) muscle(s) du membre (la jambe, le bras ou le cou, respectivement) ;
- une unité d'acquisition de signaux analogiques (9) capable d'acquérir les signaux analogiques provenant des électrodes électromyographiques et du dispositif haptique (6) et convertissant lesdits signaux analogiques en signaux numériques ;
- un contrôleur (8) capable de traiter lesdits signaux numériques et de commander l'unité mobile (1).

2. Dispositif médical selon la revendication 1, **caractérisé en ce que** le dispositif mobile est entraîné par un moteur (7) sous le contrôle du contrôleur (8),

3. Dispositif médical selon la revendication 1 ou 2, **caractérisé en ce que** les électrodes électromyographiques sont des électrodes d'aiguille et/ou des électrodes de surface.

4. Dispositif médical selon l'une quelconque des revendications précédentes, **caractérisé en ce qu'**il comprend quatre électrodes d'aiguille électromyographiques et deux électrodes actives en surface électromyographiques.

5. Dispositif médical selon la revendication 1 ou 2, **caractérisé en ce que** les électrodes électromyographiques sont des microélectrodes.

6. Dispositif médical selon l'une quelconque des revendications précédentes, **caractérisé en ce que** l'unité mobile (1) présente au moins un axe de rotation (11) autour duquel l'unité mobile (1) peut tourner par rapport au dispositif haptique (6) sous un angle de rotation (α) compris entre environ +45° et environ -45° et à une vitesse de rotation maximale définie.

7. Dispositif médical selon la revendication 6, **caractérisé en ce que** la vitesse de rotation maximale de l'unité mobile (1) est d'environ 300°/s.

8. Dispositif médical selon la revendication 6 ou 7, **caractérisé en ce que** l'unité mobile (1) présente une précision de position d'environ 0,1 mm.

9. Dispositif médical selon l'une quelconque des revendications précédentes, **caractérisé en ce qu'**il comprend en outre un système d'amplification pour amplifier les signaux analogiques mesurés par les électrodes (4).

10. Dispositif médical selon la revendication 1 ou 2, **caractérisé en ce que** le contrôleur maintient en direction et en intensité la force d'entraînement du moteur (7) et maintient ainsi en direction et en intensité le mouvement de l'unité mobile (1).
